# EUROPÄISCHE PATENTSCHRIFT

(11) **EP 1 392 190 B1**
(45) Veröffentlichungstag und Bekanntmachung des Hinweises auf die Patenterteilung: **23.08.2006**
(21) Anmeldenummer: 02743066.9
(22) Anmeldetag: 21.05.2002
(51) Int. Cl.: A61B 17/70, A61B 17/88

(54) **MANIPULATOR ZUR HANDHABUNG EINER PEDIKELSCHRAUBE**
MANIPULATOR FOR HANDLING A PEDICULE SCREW
MANIPULATEUR POUR MANIER UNE VIS PEDICULAIRE

(30) Priorität: 21.05.2001 DE 10125717
(43) Veröffentlichungstag der Anmeldung: 03.03.2004
(73) Patentinhaber: Karl Storz GmbH & Co. KG, 78532 Tuttlingen (DE)
(72) Erfinder: TIEBER, Friedrich, 86609 Donauwörth (DE)
(74) Vertreter: Weller, Wolfgang
(86) Internationale Anmeldenummer: PCT/EP2002/005565
(87) Internationale Veröffentlichungsnummer: WO 2002/094114

(56) Entgegenhaltungen:
- EP-A- 0 465 158
- WO-A-94/26190
- US-A- 5 474 558
- US-A- 5 505 732

## Beschreibung

Die Erfindung betrifft einen Manipulator mit einem stabförmigen Körper, wobei der Manipulator zur Handhabung einer eine Fixierschraube aufweisenden Pedikelschraube in einem Wirbelkörper einer Wirbelsäule dient, wobei ein Kopf der Fixierschraube konturiert ist, der stabförmige Körper eine Schraube aufweist, der stabförmige Körper ein distales Ende aufweist, das konturiert ist, so dass das distale Ende an den konturierten Kopf der Fixierschraube ansetzbar und drehschlüssig und lösbar mit dieser verbindbar ist, der stabförmige Körper mittels dessen Schraube zeitweilig mit der Fixierschraube verschraubbar ist, und wobei der Manipulator Kupplungsmittel zum Kuppeln des stabförmigen Körpers mit einem stabförmigen Werkzeug aufweist.

Ein derartiger Manipulator ist aus der WO 94 26190 A bekannt.

Pedikelschrauben werden bei einer Operationstechnik eingesetzt, die eine dreidimensionale Korrektur von Fehlstellungen der Wirbelsäule zum Ziel hat.

Diese Operationstechnik unter Einsatz von Pedikelschrauben ist beispielsweise in dem Artikel Adolf Müller et al. "A Keyhole Approach for Endoscopically Assisted Pedicle Screw Fixation in Lumbar Spine Instability", Neurosurgery, Volume 47, No. 1, Juli 2000, Seiten 85-96 beschrieben.

Sollen beispielsweise zwei benachbarte Wirbel einer Wirbelsäule komprimiert werden, also zusammengedrückt werden, wird zunächst in jeden der beiden Wirbel beidseits der posterioren Mittellinie je eine Pedikelschraube gesetzt.

Eine solche bekannte Pedikelschraube weist einen Schaft mit einem Außengewinde auf, über das die Pedikelschraube in einer zuvor präparierten Öffnung in den Wirbel eingedreht werden kann. Im Kopf der Pedikelschraube ist ein Schlitz ausgespart, in den ein fester Stab eingelegt werden kann. Dieser Stab wird zugleich in den entsprechenden Schlitz in der Pedikelschraube am benachbarten Wirbel eingelegt. Anschließend wird auf den Schraubenkopf eine Art Klemmhülse aufgeschoben, die mittels einer Fixierschraube so mit dem im Kopf aufgenommenen Stab verklemmt wird, daß dieser fest sitzt. Bei dem zuvor erwähnten Vorgang der Komprimierung zweier benachbarter Wirbel wird die Fixierschraube zunächst noch relativ locker angezogen. Die Wirbel werden dann in die gewünschte Position verschoben, also beim Komprimieren aufeinanderzubewegt, wobei sich dann die Pedikelschrauben der beiden benachbarten Wirbel aufeinanderzubewegen, und zwar längs des zwischen diesen beiden eingelegten Stabes. Anschließend wird die Fixierschraube festgezogen, so daß dann die beiden benachbarten Wirbel in ihrer neuen ausgerichteten Position verbleiben. Dies wird an beiden Pedikelschraubenpaaren beidseits der posterioren Mittellinie durchgeführt.

Es müssen also Manipulationen an den bereits gesetzten Pedikelschrauben durchgeführt werden, um das eigentlich durchzuführende Korrekturergebnis an der Wirbelsäule zu erzielen.

Es besteht nun ein allgemeines Bestreben in der Operationstechnik, Eingriffe minimal-invasiv durchzuführen.

Dazu ist in dem eingangs erwähnten Artikel in Neurosurgery beschrieben, an den vier Stellen, an denen die vier Pedikelschrauben gesetzt werden sollen, nur kleine Incisionen von wenigen Zentimetern Länge zu machen, dort entsprechende Trokare in den Körper einzubringen und dann das Setzen der Pedikelschraube durch die Hohlkörper der Trokare hindurch zu bewerkstelligen. Es ist dann noch eine weitere Incision erforderlich, die ein seitliches Einschieben der Verbindungsstäbe in die Schlitze der Pedikelschrauben ermöglicht.

Bei einer nicht minimal-invasiven Operation erfolgt eine Längsincision entlang der posterioren Mittellinie in Höhe des betroffenen Segments der Wirbelsäule. Weiterhin muß das subkutane Gewebe und die Faszie bis zur Spitze des Dornfortsatzes dissektiert werden. Die paraspinalen Muskeln müssen vom Dornfortsatz den Wirbelbögen und den Querfortsätzen der Wirbel abgelöst werden. Die Kapsel und die Gelenkknorpel der von der Fusion betroffenen Gelenke müssen entfernt werden.

Bei der minimal-invasiven Operationstechnik steht allerdings für die Manipulationen an den bereits gesetzten Pedikelschrauben nur ein relativ eingeschränkter Raum zur Verfügung, nämlich der lichte Innenraum der Trokare.

Bei der eigentlichen Korrektur der Wirbelsäule müssen erhebliche Kräfte aufgebracht werden, um die Wirbel in ihrer Lage zu verändern. Dementsprechend muß der Kopf einer Pedikelschraube voluminös ausgebildet sein, damit die entsprechenden Manipula-, tionen überhaupt durchgeführt werden können. Dabei ist zu bedenken, daß bei minimal-invasiven Eingriffen das vom Operateur ersichtliche Operationsfeld äußerst gering ist und der Operateur oftmals Schwierigkeiten hat, den korrekten Sitz eines Werkzeugs an der Pedikelschraube zu überprüfen.

Da die Pedikelschraube nach dem Eingriff im Körper verbleibt, besteht die Möglichkeit, daß durch einen voluminösen, insbesondere vom Wirbel weit abstehenden Schraubenkopf postoperative Traumata erzeugt werden.

Die eingangs erwähnte WO 94 26190A zeigt einen zweiteiligen Manipulator für eine Pedikelschraube, bestehend aus einer zylindrischen Hülse und einem zylindrischen Stift, der in die Hülse eingeführt werden kann und in der Hülse sowohl drehbar als auch axial beweglich ist. Die Hülse weist an ihrem unteren distalen Ende eine Kontur auf, die in eine entsprechend negativ konturierte Aussparung (Nut) eines Verschlußteils drehschlüssig eingreifen kann. Über den drehschlüssigen Eingriff kann das Verschlußteil mit dem als Hülse ausgebildeten Manipulator in eine Pedikelschraube eingedreht werden. Dazu weist das Verschlußteil ein Außengewinde und die Pedikelschraube ein Innengewinde auf.

Das Verschlußteil ist axial durchbohrt und besitzt in der Bohrung ein Innengewinde. In dieses Innengewinde wird der in der Manipulator-Hülse beweglich geführte zylindrische Stift mit seinem unteren Ende eingeschraubt. Der mit dem Verschlußteil verschraubte zylindrische Stift bildet damit eine Führung für die auf dem Stift axial bewegliche und drehbare Manipulator-Hülse. Durch axiales Verschieben der Hülse auf dem Stift kann die drehschlüssige Verbindung der Hülse mit dem Verschlußelement gelöst werden. Die axiale Führung erleichtert das drehschlüssige Aussetzen der Hülse auf das Verschlußelement der Pedikelschraube.

Aus der US-A-6,224,598 B1 ist ein Verschlußelement mit Klemmschraube für eine Pedikelschraube bekannt. Das Verschlußelement ist als Hülse mit einem Außengewinde und einem Innengewinde realisiert. Abgesehen von den beiden genannten Gewinden ist diese Hülse rotationssymmetrisch. Die Hülse wird mit ihrem Au-ßengewinde in ein korrespondierendes Innengewinde einer Pedikelschraube eingedreht. Dazu wird die Hülse zunächst mit ihrem Innengewinde auf ein korrespondierendes Außengewinde eines Drehwerkzeuges geschraubt. Anschließend wird die Hülse mit dem Drehwerkzeug in die Pedikelschraube eingedreht. Während die Hülse in der Pedikelschraube verbleibt, wird das Drehwerkzeug wieder ausgedreht und in das dadurch freiwerdende Innengewinde der Hülse wird eine Klemmschraube eingeschraubt. Im eingeschraubten Zustand klemmen die Hülse und die in der Hülse eingeschraubte Klemmschraube eine Stange in der Pedikelschraube ein. Wenn sich die Stange in der gewünschten Endposition befindet, wird die Klemmschraube weiter gedreht, bis ihr Sechskant an einer Sollbruchstelle abreißt. Hinweise darauf, wie ein Manipulator an der Pedikelschraube, dem Verschlußteil oder der Klemmschraube angreift, sind dieser Schrift nicht zu entnehmen.

Mit Blick auf den oben dargestellten Stand der Technik besteht die Aufgabe der Erfindung darin, einen Manipulator bereitzustellen, der eine verbesserte Handhabung einer Pedikelschraube in einem Wirbelkörper einer Wirbelsäule, insbesondere bei minimal-invasiven Operationstechniken, ermöglicht.

Die Aufgabe wird erfindungsgemäß dadurch gelöst, dass die Schraube im Inneren des stabförmigen Körpers aufgenommen ist, und dass im stabförmigen Körper ein Gewinde eingeschnitten ist, in das ein Gewinde der Schraube eindrehbar und durch dieses hindurch drehbar ist.

Das Vorsehen eines zwischen die Pedikelschraube und dem eigentlichen Werkzeug zwischengeschalteten Manipulators ermöglicht, diesen entsprechend stabil und mit einer entsprechenden Länge aufzubauen, die ein sicheres Heranführen und Ansetzen eines Werkzeuges und die entsprechenden Kraft übertragenden Manipulationen erlaubt. Die Fixierschraube der Pedikelschraube ist ohnehin ein relativ stabiles Bauteil, das dazu dient, den in den Schlitz im Kopf der Pedikelschraube eingelegten Stab zu fixieren. Die Schaffung der Möglichkeit, diese Fixierschraube lösbar, jedoch drehschlüssig und fest am distalen Ende des Manipulators anzubringen, eröffnet nun die Möglichkeit, die bei der eigentlichen Wirbelkorrektur notwendigen Manipulationsmaßnahmen in sicherer und einfacher Weise durchzuführen.

Nach Setzen der Pedikelschraube, Einlegen des Stabes und Aufsetzens einer Klemmhülse kann nun ein sicher handhabbares und kompakt ausgebildetes, jedoch gleichzeitig schlank gebautes Werkzeug herangeführt werden. Dieses Werkzeug besteht aus dem Manipulator und dem über dessen Kupplungsmittel mit diesem verkuppelten stabförmigen Werkzeug.

In einem ersten Arbeitsgang kann durch dieses insgesamt schlank bauende Werkzeug die Fixierschraube eingedreht werden, jedoch nur so fest, daß Manipulationen noch möglich sind, d.h. also die Köpfe der Pedikelschraube längs des Stabes bei der eigentlichen Korrektur verschoben werden können. Dazu kann der Manipulator, der nach wie vor mit der Fixierschraube der Pedikelschraube drehschlüssig und fest verbunden ist, an Ort und Stelle verbleiben und es kann auf den schaftartig von dem Kopf der Pedikelschraube hochstehenden stabförmigen Körper des Manipulators ein anderes Werkzeug, beispielsweise das Repositionswerkzeug, aufgesetzt werden. Durch Vorsehen der Kupplungsmittel kann der Manipulator jeweils mit dem entsprechenden Werkzeug verkuppelt werden. Der Manipulator dient quasi als Zwischenstück oder Hilfsinstrument, das es dem Operateur wesentlich erleichtert, insbesondere bei minimal-invasiven Operationstechniken die entsprechenden Manipulationen durchzuführen. Es ist für den Operateur wesentlich einfacher, auf den mit einer entsprechenden Länge ausgestatteten stabförmigen Körper des Manipulators Werkzeuge aufzuschieben bzw. abzunehmen, als wenn er das direkt an dem tief im Körper sitzenden Pedikelschraubenkopf vornehmen müßte. Die Länge kann so gewählt werden, daß das proximale Ende vom Operateur problemlos ersichtlich ist, er also dann problemlos ein Werkzeug aufschieben kann. Ein weiterer beachtlicher Vorteil ist die Möglichkeit eines sogenannten Low-Profile-Design des Schraubenkopfes. Daraus resultieren geringere Schädigungen von Weichteilen, Gefäßen und knöcherner Substanz. Dieses Design reduziert post-operative Schmerzen und Sensitivität.

Ist die Korrektur der Wirbel durchgeführt worden, kann die Verbindung zwischen dem Manipulator und der mit diesem immer noch fest verbundenen Fixierschraube wieder gelöst werden. Dabei bleibt aber selbstverständlich die Fixierschraube der Pedikelschraube fest auf deren Kopf aufgedreht, so daß das Hilfsgerät in Form des Manipulators abgenommen werden kann.

Durch die schlanke stabförmige Bauweise können nun alle erforderlichen Manipulationen nach Setzen der Pedikelschraube von dem Operateur in einfacher Art und Weise durch relativ durchmessergeringe Trokare hindurch durchgeführt werden, und zwar unter Einsatz von den entsprechenden Low-Profile-Design Pedikelschrauben.

Insgesamt gesehen wird durch den Manipulator nicht nur die Handhabung für den Operateur vereinfacht und erleichtert, sondern an dem Patienten selbst sind wesentlich weniger tiefe Eingriffe notwendig, und durch das Low-Profile-Design der Schraubenköpfe sind die post-operativen Schmerzen, Sensivitäten oder auch Schädigungen stark reduziert.

Das feste Verbinden des Manipulators mit der Fixierschraube durch eine Schraube hat den Vorteil, daß die Schraube ein sehr schlank bauendes Mittel ist, das am schlank bauenden Manipulator vorhanden ist.

Weiter können durch den unverlierbaren festen Sitz des Manipulators an der Fixierschraube die jeweiligen Flächen, über die die Drehkräfte von dem Werkzeug auf die Fixierschraube übertragen werden, verringert werden.

Flach ausgeführte drehschlüssige Steckverbindungen weisen im allgemeinen den Nachteil auf, daß übliche. Drehwerkzeuge, die ohne axiale Fixierung bspw. auf einen Innen- oder Außensechskant gesteckt werden, bereits bei leichten Verkippungen von dem Seckskant abrutschen. Dies gilt insbesondere dann, wenn vergleichsweise hohe Drehmomente zu übertragen sind, wie es beim festen Anziehen einer Fixierschraube der Fall ist. Um dies zu vermeiden, sind die üblichen Eingriffsflächen drehschlüssiger Verbindungen vergleichsweise hoch ausgeführt.

Vor diesem Hintergrund besteht ein besonderer Vorteil der Erfindung darin, daß die Verschraubung der Fixierschraube mit dem Manipulator beide Elemente relativ zueinander axial fixiert, was eine flache Ausführung der drehschlüssigen Verbindung auch bei hohen zu übertragenden Drehmomenten ohne die Gefahr eines Abrutschens des Drehwerkzeuges ermöglicht.

Im Zusammenhang mit den oben ausführlich dargestellten medizinischen und operationstechnischen Randbedingungen ergibt sich dadurch als beachtlicher Vorteil die Möglichkeit der Verwendung eines sog. *low-profile-designs* des Schraubenkopfes mit geringen Höhen der zwangsläufig mit Kanten versehenen Drehschluß-Eingriffsflächen an der Fixierschraube. Daraus resultieren geringere Schädigungen von Weichteilen, Gefäßen und knöcherner Substanz. Dieses Design reduziert post-operative Schmerzen und Sensitivität.

Dadurch, daß der Kopf der Fixierschraube konturiert ist und das distale Ende des stabförmigen Körpers des Manipulators komplementär konturiert ist, wodurch der Kopf form- und drehschlüssig am distalen Ende aufnehmbar ist, wird der Form-, Kraft- und Drehschluß schon durch die entsprechende Konturierung bewerkstelligt. Die Schraube des Manipulators dient lediglich zur unverlierbaren festen Halterung und muß keine großen Kräfte übertragen. Dadurch kann diese Schraube relativ schlank und einfach ausgebildet werden.

Bei der Erfindung ist die Schraube im Inneren des stabförmigen Körpers aufgenommen.

Diese Maßnahme hat den Vorteil, daß die Außenkontur des stabförmigen Körpers relativ durchmessergroß ausgebildet werden kann, dieser somit eine ,entsprechende Stabilität aufweist, und die Schraube zum Verbinden mit der Fixierschraube ist im Inneren des Körpers aufgenommen.

Bei der Erfindung ist im stabförmigen Körper ein Gewinde eingeschnitten, in das ein Gewinde der Schraube eindrehbar und durch diesen hindurch drehbar ist.

Diese Maßnahme hat den Vorteil, daß die beiden Bauteile des Manipulators über das Gewinde miteinander verbunden werden können und somit die Schraube unverlierbar am Manipulator gehalten ist. Zum Reinigen oder Desinfizieren nach einem Einsatz kann die Schraube ausgedreht werden, zum Verbinden mit der Fixierschraube kann dann die Schraube weiter in das Gewinde im Manipulator hinein oder auch durch das Gewinde hindurch am distalen Ende hinausgedreht werden, je nachdem, wie die Konstruktion der Fixierschraube der Pedikelschraube ist.

In einer weiteren Ausgestaltung der Erfindung ist in der Fixierschraube ein Gewinde vorhanden, in die das Gewinde der Schraube eindrehbar ist.

Diese Maßnahme hat den Vorteil, daß durch einfache Mittel die Fixierschraube der Pedikelschraube mit dem Manipulator verbunden werden kann.

In einer weiteren Ausgestaltung der Erfindung weisen die Kupplungsmittel einen Mehrkant auf, der mit einem entsprechenden Mehrkant am stabförmigen Werkzeug in kuppelnden Eingriff steht, wenn das Werkzeug auf den stabförmigen Körper aufgeschoben ist.

Durch diese Ausgestaltung ist es möglich, auch bei relativ schlanken Körpern einen relativ großen flächigen Eingriff beim Kuppeln zu bewerkstelligen, über den die Drehkräfte gleichmäßig verteilt werden können. Dies ist insbesondere bei der minimal-invasiven Operationstechnik von besonderem Vorteil.

In einer weiteren Ausgestaltung der Erfindung ist der Mehrkant als Außensechskant am stabförmigen Körper ausgebildet.

Diese Maßnahme hat den Vorteil, daß dies als für den Operateur leicht ersichtliches Orientierungsmerkmal dient. Dadurch ist es für den Operateur einfach, auf diesen Außensechskant ein entsprechendes Werkzeug aufzuschieben.

In einer weiteren Ausgestaltung der Erfindung weisen die Kupplungsmittel Rastmittel auf, um den Manipulator mit dem aufgesetzten stabförmigen Werkzeug zu verrasten.

Diese Maßnahme hat den Vorteil, daß ein aufgesetztes Werkzeug gegen axiales Lösen durch die Rastmittel gesichert ist.

In einer weiteren Ausgestaltung der Erfindung sind die Rastmittel als Kugelraste ausgebildet.

Diese Maßnahme hat den Vorteil, daß das Ein- und Ausrasten über eine Kugelraste sanft und insbesondere für den Patienten atraumatisch durchzuführen ist.

In einer weiteren Ausgestaltung der Erfindung weisen die Rastmittel eine Aussparung am stabförmigen Körper auf, in die eine federbelastete Kugel des Werkzeuges einrastbar ist.

Diese Maßnahme hat den Vorteil, daß durch konstruktiv einfache und die Stabilität des Manipulators nicht beeinträchtigende Mittel eine ausreichend feste Verrastung bewerkstelligt werden kann.

In einer weiteren Ausgestaltung der Erfindung weist der stabförmige Körper am distalen Endbereich einen ersten zylindrischen Abschnitt auf, an den sich nach proximal ein Au-ßensechskant anschließt, dessen Kreisumfangslinie einen geringeren Durchmesser als der erste zylindrische Abschnitt aufweist, und daran schließt sich ein zweiter zylindrischer Abschnitt mit einem noch geringeren Durchmesser an.

Diese Maßnahme hat den Vorteil, daß durch die Abstufung auf den Manipulator ein Werkzeug aufgesetzt werden kann, dessen Außendurchmesser nicht größer als der Außendurchmesser des ersten zylindrischen Abschnittes ist. Dadurch ist dann der Zusammenbau aus Manipulator und Werkzeug äußerst schlank und es können dann auch Manipulationen, wie Kippbewegungen innerhalb einer Trokarhülse, durchgeführt werden.

Es versteht sich, daß die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Die Erfindung wird nachfolgend anhand der beiliegenden Zeichnungen näher beschrieben und erläutert.

Es zeigen:
- Fig. 1: einen Längsschnitt durch einen erfindungsgemäßen Manipulator in Explosionsdarstellung,
- Fig. 2: einen Schnitt längs der Linie II-II in Fig. 1,
- Fig. 3: einen Schnitt längs der Linie III-III in Fig. 1,
- Fig. 4: eine dem Schnitt von Fig. 1 vergleichbare Darstellung eines erfindungsgemäßen Manipulators und einer Explosionsdarstellung einer Pedikelschraube, die mit dem Manipulator gehandhabt werden soll,
- Fig. 5: einen Schnitt durch einen Wirbel, in den zwei Pedikelschrauben eingesetzt sind,
- Fig. 6: eine Seitenansicht einer Wirbelsäule, in der an drei aufeinanderfolgenden Wirbelkörpern jeweils eine Pedikelschraube eingesetzt sind, wobei dargestellt ist, wie gerade ein diese drei Pedikelschrauben verbindender Stab mittels eines Stabsetzwerkzeuges eingesetzt wurde,
- Fig. 7: einen Manipulator gekoppelt mit einem stabförmigen Werkzeug zur Manipulation an einer in einem Wirbelkörper gesetzten Pedikelschraube,
- Fig. 8: einen anderen Betriebszustand des Manipulators, wie er an einen Kopf einer gesetzten Pedikelschraube befestigt ist,
- Fig. 9: eine Seitenansicht einer Wirbelsäule, wobei dargestellt ist, wie ein Repositionsgerät auf zwei erfindungsgemäße Manipulatoren aufgesetzt ist,
- Fig. 10: eine stark schematische Schnittdarstellung über die Möglichkeit einer Verbindung zweier Pedikelschrauben über einen Querverbinder,
- Fig. 11: eine Draufsicht auf den Querverbinder von Fig. 10, und
- Fig. 12: eine endfertig gesetzte Pedikelschraube nach Abschluß aller Manipulationen.

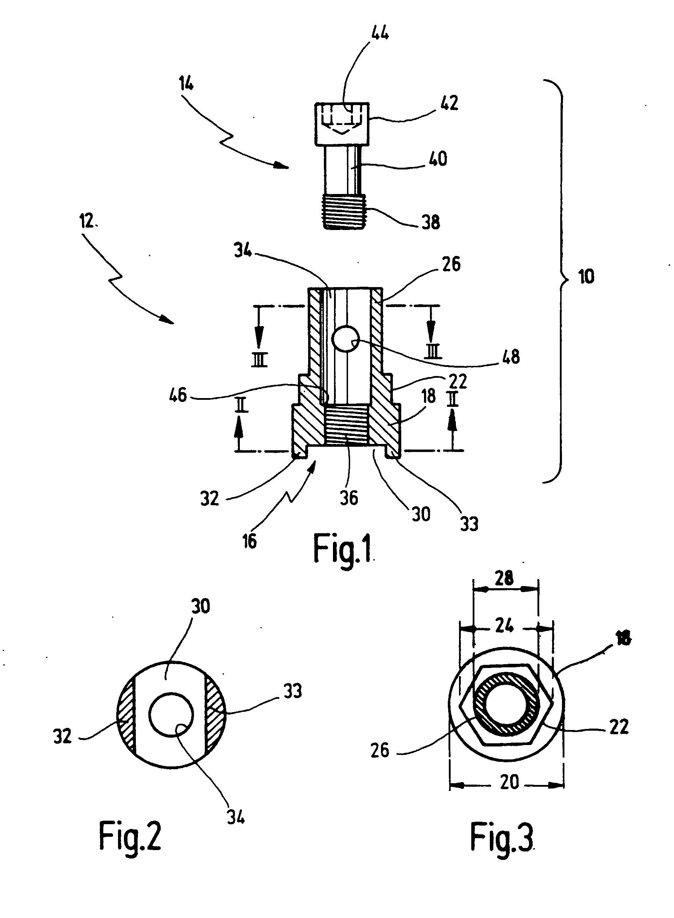

Ein in Fig. 1 dargestellter Manipulator ist in seiner Gesamtheit mit der Bezugsziffer 10 bezeichnet.

Der Manipulator 10 besteht aus einem etwa stabförmigen Körper 12 und einer Schraube 14.

Im Bereich eines distalen Endes 16 weist der stabförmige Körper 12 einen ersten zylindrischen Abschnitt 18 mit einem Durchmesser 20 auf, wie das insbesondere aus der Draufsicht von Fig. 3 ersichtlich ist. Nach proximal gesehen schließt sich an den ersten zylindrischen Abschnitt 18 ein Außensechskant 22 an, dessen maximaler Außendurchmesser 24 geringer ist als der Durchmesser 20 des ersten zylindrischen Abschnittes 18.

An den Außensechskant 22 schließt sich ein zweiter zylindrischer Abschnitt 26 an, dessen Durchmesser 28 nochmals geringer ist als der Durchmesser 24 des Außensechskant 22. Die Länge des zweiten zylindrischen Abschnitts 26 ist etwa doppelt so groß wie die axiale Länge des ersten zylindrischen Abschnitts 18 und die axiale Länge des Außensechskantes 22.

In der Stirnseite des distalen Endes 16 ist eine Aussparung 30 vorhanden, die so geformt ist, daß beidseits der Aussparung noch Abschnitte in Form von Kreissegmenten 32 und 33 stehen bleiben, wie das insbesondere aus Fig. 2 in Zusammenhang mit Fig. 1 ersichtlich ist.

Durch den stabförmigen Körper 12 geht mittig eine durchgehende Öffnung 34 hindurch, die im Bereich des ersten zylindrischen Abschnittes 18 mit einem Gewinde 36 in Form eines Innengewindes versehen ist.

Die Schraube 14 weist an ihrem distalen Ende ein Gewinde 38 in Form eines Außengewindes auf, das passend in das Innengewinde 36 im Innern des stabförmigen Körpers 12 eingedreht und auch durch dieses hindurchgedreht werden kann. Die Schraube 14 weist nach proximal gesehen von dem Gewinde 38 vorstehend einen Schaft 40 auf, der in einen erweiterten Kopf 42 mündet. Im Kopf 42 ist ein Innensechskant 44 ausgespart.

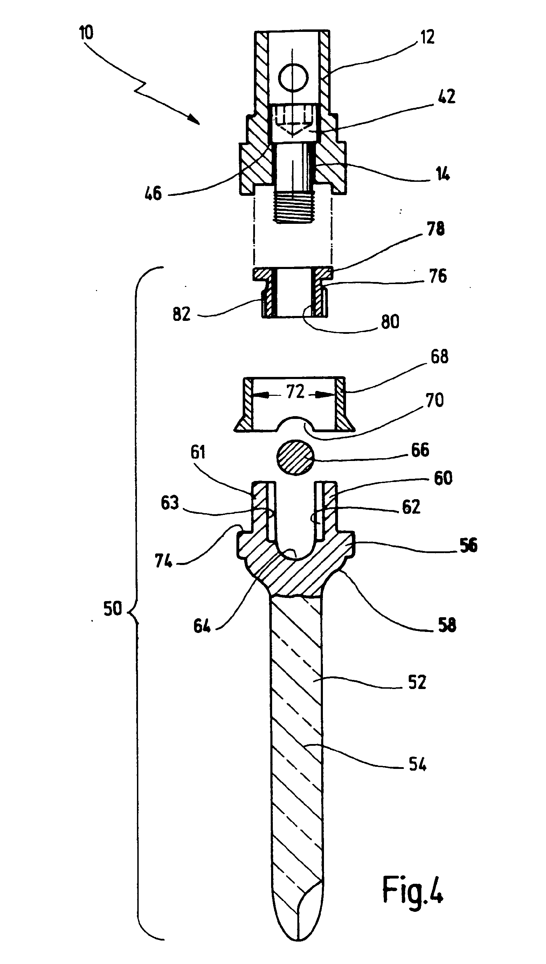

Die durchgehende Öffnung 34 im Innern des stabförmigen Körpers 12 geht über eine Ringschulter 46 in das Innengewinde 36 über, wobei der Kopf 42 auf dieser nach innen vorspringenden Ringschulter 46 anliegt, wenn die Schraube 14 maximal weit nach innen eingeschoben ist, wie das am oberen Ende von Fig. 4 ersichtlich ist.

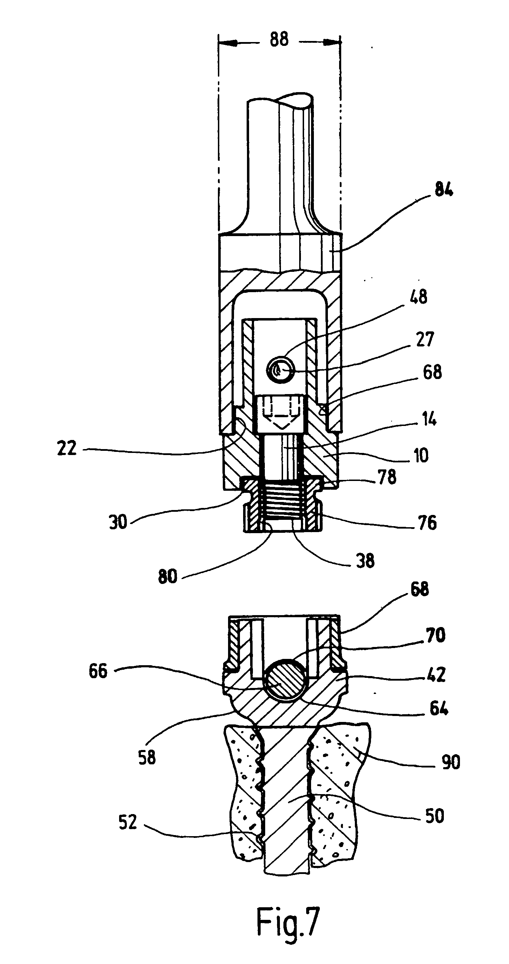

Im Zylindermantelkörper des zweiten zylindrischen Abschnitts 46 des Manipulators 10 ist noch eine Aussparung 48 in Form einer seitlichen durchgehenden Bohrung vorgesehen. Diese Aussparung 48 dient dazu, daß in diese eine Kugel einer Kugelraste eintreten kann, wie das später noch nachfolgend in Zusammenhang mit Fig. 7 beschrieben wird.

Wie bereits zuvor erwähnt, dient der Manipulator 10 dazu, eine Pedikelschraube 50, wie sie in Fig. 4 in Explosionsdarstellung dargestellt ist, zu handhaben.

Die Pedikelschraube 50 weist einen lang erstreckten Schaft 52 auf, der mit einem Außengewinde 54 versehen ist. Am proximalen Ende ist der Schaft 52 mit einem Kopf 56 versehen, der etwas erweitert ist und eine sanft gekrümmte Unterseite 58 aufweist.

Von der Oberseite des Kopfes 56 stehen zwei diametral gegenüberliegende Zylindermantelabschnitte 60 bzw. 61 axial nach oben vor, die jeweils mit einem Innengewinde 62 bzw. 63 versehen sind. Zwischen den Zylindermantelabschnitten 60, 61 ist im Boden des Kopfes 56 noch eine halbrunde Mulde 64 vorgesehen, in die ein Stab 66 eingelegt werden kann.

Der Sinn und Zweck des Stabes 66 wird später noch in Zusammenhang mit der Handhabung beschrieben.

Zur Festlegung des Stabes 66 ist ein Preßring 68 vorgesehen, der so ausgebildet ist, daß dessen lichter Innendurchmesser 72 in etwa dem Außendurchmesser der äußeren Fläche der Zylindermantelabschnitte 60, 61 entspricht. An zwei diametral gegenüberliegenden Seiten der distalen unteren Kante des Preßringes 68 sind zwei halbrunde Aussparungen 70, 71 vorgesehen, die der Rundung des Stabes 66 angepaßt sind. Eine Ringschulter 74 am Kopf 56 der Pedikelschraube 50 dient als Widerlager für den aufgeschobenen Preßring 68.

Zur Fixierung des Zusammenbaus aus Kopf 56 der Pedikelschraube 50, eingelegtem Stab 66 und aufgeschobenem Preßring 68 ist eine Fixierschraube 76 vorgesehen.

Die Fixierschraube 76 weist einen Kopf 78 auf, dessen Kontur der Negativkontur der Aussparung 30 am distalen Ende des Manipulators 10 entspricht. Anders ausgedrückt ist der Kopf 78 eine Scheibe, bei der an diametral gegenüberliegenden Seiten entsprechende Kreissegmente abgeschnitten wurden, wobei diese Segmente den Segmenten 32 und 33 am distalen Ende des Manipulators 10 entsprechen. Die Höhe des Kopfes 78 entspricht etwa der Tiefe der Aussparung 30 am distalen Ende des Manipulators 10, so daß der Kopf 78 der Fixierschraube 76 form- und demzufolge auch kraft- und drehschlüssig in das distale Ende 16 des Manipulators 10 eingelegt werden kann.

Wie aus Fig. 4 zu entnehmen, ist die Fixierschraube 76 mit einem durchgehenden Innengewinde 80 versehen, wobei dieses Innengewinde 80 so ausgebildet ist, daß darin das Außengewinde der Schraube 14 des Manipulators 10 eingedreht werden kann.

Zusätzlich ist die Fixierschraube 76 noch mit einem Außengewinde 82 versehen, wobei das Außengewinde 82 so ausgebildet ist, daß es in die Innengewinde 62, 63 der vom Kopf 56 hochstehenden Zylindermantelabschnitte 60, 61 eingedreht werden kann.

Die weiteren Bauteile der Pedikelschraube 50 bzw. deren Zusammenwirken sollen nachfolgend anhand der Operationstechnik näher beschrieben werden.

Zunächst muß der Eintrittspunkt der Pedikelschraube bestimmt werden. Dazu identifiziert man den Kammansatz am unteren Punkt des Gelenkes des superioren Wirbels. Der Insertionspunkt befindet sich geringfügig außerhalb der Gelenklinie des knöchernen Kammes, gegenüber der Basis des Querfortsatzes. Die korrekte konvergente Richtung entlang der Mittellinie der Pedikelachse wird durch Fühlen mittels eines Spatels des äußeren kortikalen Knochens bestimmt. Bei der minimal-invasiven Technik wird der Zugang zum Eintrittspunkt jeweils über einen Trokar bewerkstelligt. Sollen also vier Pedikelschrauben eingesetzt werden, werden an den entsprechenden Stellen vier Trokare in an sich bekannter Art und Weise gesetzt.

Mit einer Reibdahle wird die Öffnung im Knochen soweit erweitert, daß darin eine Pedikelschraube gesetzt werden kann.

Röntgenfähige Führungsdrähte werden in die Pedikelkanäle eingesetzt, je nach Operationstechnik bis zur Gegenkortikalis des Wirbelkörpers. Die Länge der zu verwendenden Pedikelschraube wird bestimmt, indem ein Meßinstrument über den Führungsdraht bis zum Eintrittspunkt geleitet wird. Die erforderliche Schraubenlänge kann nun an der Skala des Meßinstrumentes abgelesen werden.

Es wird eine entsprechende Pedikelschraube gefaßt und die Schraube wird entsprechend der Ausrichtung des Führungsdrahtes in dem Pedikel soweit wie möglich eingedreht.

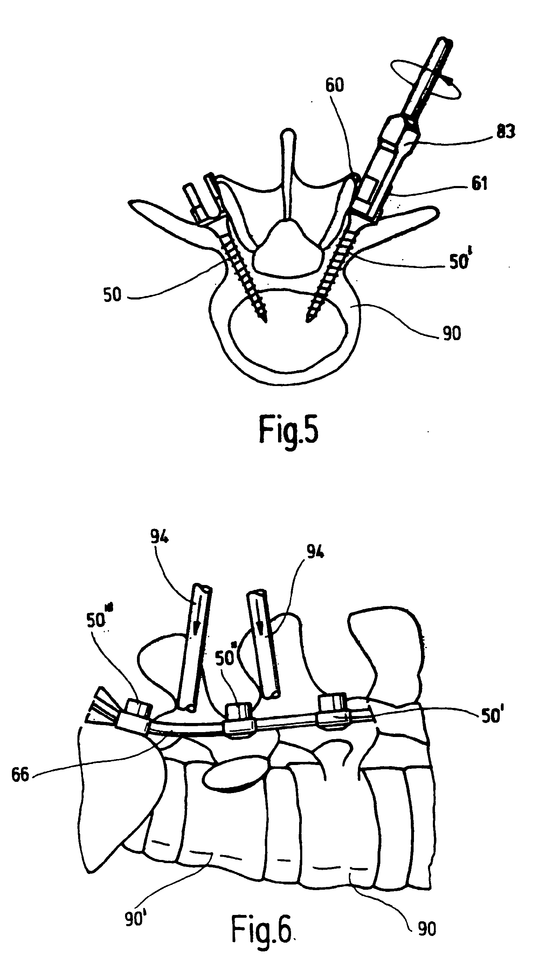

In Fig. 5 ist nun eine Situation dargestellt, bei der bereits eine Pedikelschraube 50 in einen Wirbel 90 gesetzt wurde, die entsprechend konvergent dazu angeordnete Pedikelschraube 50' an der gegenüberliegenden Seite der Mittellängslinie wird gerade mittels eines Schraubendrehers 83 eingedreht. Dazu greift der Schraubendreher 83 in den Schlitz zwischen die beiden hochstehenden Zylindermantelabschnitte 60, 61 am Kopf 56 der Pedikelschraube 50' ein.

Entsprechende Paare an Pedikelschrauben 50, 50' werden noch an den zwei nachfolgenden wirbeln gesetzt.

Diese Situation ist in Fig. 6 dargestellt, d.h. es sind am Wirbel 90 das Pedikelpaar 50, 50' gesetzt, wobei in dieser Seitenansicht die Pedikelschraube 50 nicht ersichtlich ist.

Am nächsten Wirbel 90' ist ein entsprechendes Pedikelschraubenpaar gesetzt, wobei die Pedikelschraube 50" ersichtlich ist.

Entsprechendes gilt für den nachfolgenden Wirbel, wobei hier dann die entsprechende Pedikelschraube 50"' ersichtlich ist.

In Fig. 6 ist nun eine Situation dargestellt, in der ein Stab 66 in die drei Schlitze zwischen den jeweiligen Zylindermantelabschnitten der Pedikelschrauben 50', 50", 50''' eingesetzt worden ist. Bei einer nicht minimal-invasiven Operationstechnik, bei der die Wirbel vollständig freigelegt sind, kann der Stab 66 von oben in die Köpfe der Pedikelschrauben 50', 50' ' , 50' ' ' eingeschoben werden, wobei hier ein entsprechendes Stabsetzwerkzeug 94 zu Hilfe genommen wird.

Bei der minimal-invasiven Operationstechnik wird eine seitliche Inzision angefertigt, über die dann der Stab 66 seitlich eingeschoben wird, wobei das Einfädeln durch die über den Köpfen der Pedikelschrauben 50, 50', 50" sitzenden Trokare beobachtet werden kann, die der Übersicht halber hier nicht dargestellt sind.

Anschließend wird jeweils ein Preßring 68 auf die Köpfe aufgeschoben.

Diese Situation ist in Fig. 7 näher dargestellt. Daraus ist ersichtlich, daß die Pedikelschraube 50 in den Wirbel 90 eingedreht ist, der Stab 66 ist in die halbrunde Munde 64 eingelegt und der Preßring 68 ist aufgeschoben, so daß dessen beide halbrunde Aussparungen 70 und 71 auf der Oberseite des Stabes 66 aufliegen. Dadurch ist bereits eine Zwei-Punkt-Fixierung des Stabes 66 im Kopf 56 der Pedikelschraube 50 gewährleistet.

Aus der oberen Hälfte von Fig. 7 in Zusammenhang mit der Explosionsdarstellung von Fig. 4 ist ersichtlich, daß zwischenzeitlich ein Zusammenbau zwischen dem Manipulator 10 (bestehend aus stabförmigen Körper 12 und darin eingesetzter Schraube 14) und der Fixierschraube 76 bewerkstelligt wurde.

Wie zuvor beschrieben, sitzt nunmehr die Fixierschraube 76 in der Aussparung 30 am distalen Ende des Manipulators 10, und zwar unverlierbar fest sitzend und drehschlüssig.

Auf die Außenseite des Manipulators 10 ist nunmehr ein stabförmiges Werkzeug 84 aufgeschoben, das an seiner Innenseite mit einem Innensechskant 86 versehen ist, der dem Außensechskant 22 entspricht. Durch die zuvor beschriebene abgestufte Ausbildung des stabförmigen Körpers 12 des Manipulators 10 kann ein solches stabförmiges Werkzeug 84 aufgesetzt werden, dessen Außendurchmesser 88 in etwa dem Außendurchmesser 20 des Manipulators 10 entspricht.

Insgesamt gesehen entsteht dabei ein sehr schlankes Bauelement, zusammengesetzt aus stabförmigem Werkzeug 84 und Manipulator 10, an dem die Fixierschraube 76 der Pedikelschraube 50 angebracht ist. Das stabförmige Werkzeug 84 ist mit einer Kugelraste versehen, wobei aus Fig. 7 ersichtlich ist, daß deren Kugel 87 in die Aussparung 48 im zweiten zylindrischen Abschnitt 26 eingerastet ist. Dadurch ist der Manipulator 10 axial unverlierbar, jedoch lösbar am stabförmigen Werkzeug 84 angebracht. Durch Eingriff des Innensechskants 86 mit dem Außensechskant 22 ist eine Verbindung geschafft, die hohe Kräfte auch bei äußerst schlanker Bauweise übertragen kann.

Die Schraube 14 des Manipulators 10 in Zusammenhang mit der Aussparung 30 am distalen Ende 16 des Manipulators stellen somit Mittel dar, um den Manipulator 10 mit der Fixierschraube 76 einer Pedikelschraube drehschlüssig, jedoch lösbar am distalen Ende anzubringen.

Der Kugelrastenmechanismus und die Aussparung 48 sowie das Zusammenwirken von Innensechskant 86 und Außensechskant 22 stellen Kupplungsmittel zum Kuppeln des stabförmigen Körpers 12 mit dem stabförmigen Werkzeug 84 dar.

Der weitere Operationsverlauf ist nunmehr so, daß der in Fig. 7 dargestellte Zusammenbau aus Werkzeug 84, Manipulator 10 und Fixierschraube 76 auf den Kopf 56 der Pedikelschraube 50 angesetzt und die Fixierschraube 76 in die Innengewinde 62 bzw. 63 der hochstehenden Zylindermantelabschnitte 60 bzw. 61 eingedreht wird. Dadurch wird der Preßring 68 auf den Stab 66 mit einer bestimmten Preßkraft eingedrückt, die einen festen Sitz ermöglicht, jedoch noch Manipulationen erlaubt, bei denen Relativverschiebungen zwischen dem Stab 66 und der Pedikelschrauben 50 in einem gewissen Maß notwendig sind.

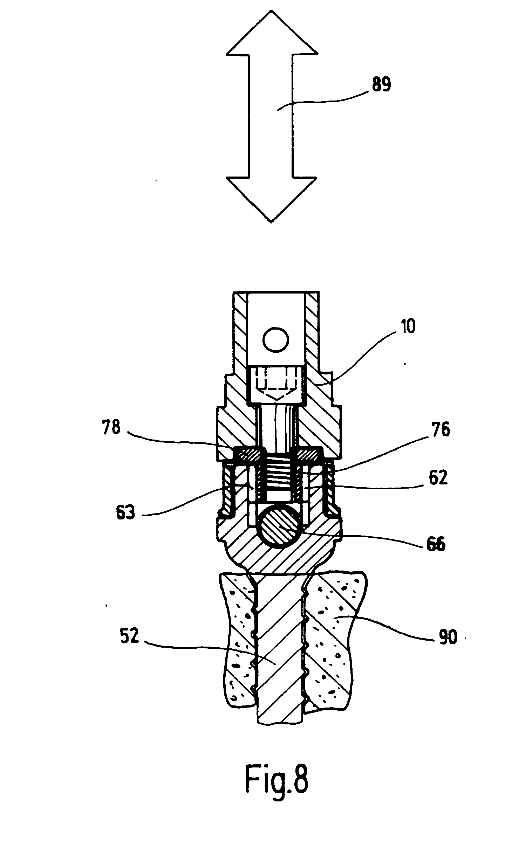

Nach Eindrehen der Fixierschraube 76 wird das stabförmige Werkzeug 64 nach oben vom Manipulator 10 abgezogen und dieser verbleibt auf dem Kopf 56 der Pedikelschraube 50, wie das in Fig. 8 dargestellt ist.

Aus Fig. 8 ist ersichtlich, daß auf der Pedikelschraube 50 der schlanke Körper des Manipulators 10 sitzt, fest mit dieser verschraubt ist, so daß über den Manipulator 10 Manipulationen an der bereits im Wirbel 90 gesetzten Pedikelschraube 50 vorgenommen werden können.

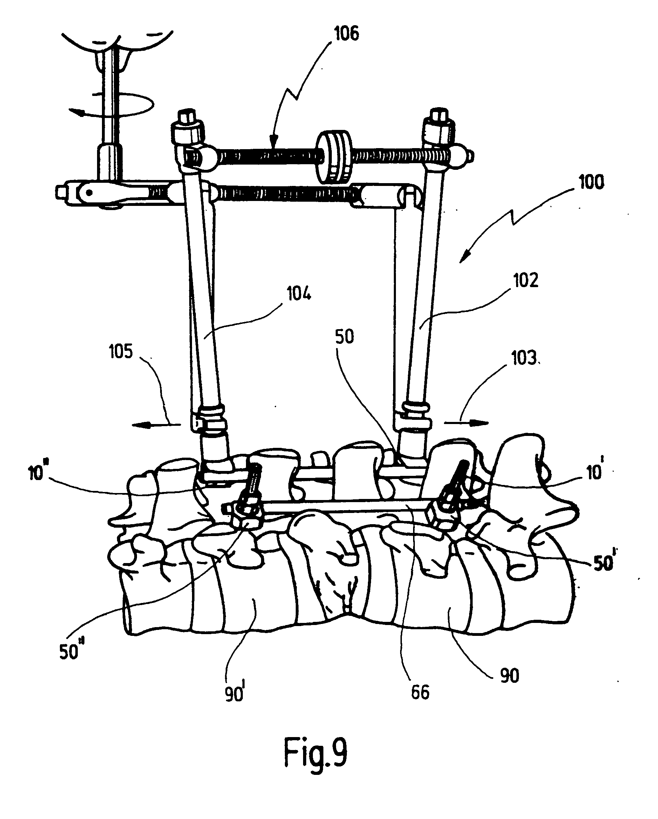

Eine solche Manipulation ist in Fig. 9 dargestellt. Daraus ist ersichtlich, daß in den Wirbel 90 diametral gegenüberliegend in einer geneigten Anordnung, wie sie aus Fig. 5 ersichtlich ist, die beiden Pedikelschrauben 50 und 50' gesetzt worden sind, daß darauf jeweils ein Manipulator 10 bzw. 10' montiert ist, wie das in Fig. 8 dargestellt ist.

Entsprechend wurden an dem Wirbel 90' zwei Pedikelschrauben 50" und eine diesen gegenüberliegende gesetzt, auf denen ebenfalls entsprechende Manipulatoren 10' ' aufgesetzt sind.

Die Pedikelschrauben 50' und 50' ' sind über den Stab 66 miteinander verbunden, dementsprechend sind dann die beiden hier nicht ersichtlichen Pedikelschrauben über einen entsprechenden Stab verbunden.

Auf die beiden hier nicht ersichtlichen Manipulatoren ist ein Repositionsgerät 100 aufgesetzt.

Das Repositionsgerät 100 weist etwa zwei stangenförmige Beine 102 und 104 auf, wobei die Stange 102 auf dem Außensechskant des entsprechenden Manipulators 10 sitzt, und die Stange 104 auf dem entsprechenden Außensechskant des Manipulators an dieser Stelle. Die Stangen 102 und 104 sind über eine Mechanik 106 verbunden, die nun die gewünschten Korrekturen erlaubt.

Die in Fig. 9 dargestellte Korrektur ist eine Distraktion der beiden Wirbel 90 und 90', d.h. diese sollen voneinander wegbewegt bzw. auseinandergezogen werden, wie das durch die Pfeile 103 und 105 dargestellt ist.

Um diese Bewegung zu ermöglichen, ist der auf der jeweiligen Pedikelschraube 50 aufgesetzte Manipulator 10 bzw. die mit diesem verbundene Fixierschraube 76 nur so fest aufgedreht, daß gerade diese Bewegungen noch möglich sind.

Wurde die Korrektur durchgeführt, werden die Fixierschrauben 76 über die mit diesen noch fest verbundenen entsprechenden Manipulatoren 10 und auf deren Außensechskant 22 aufgesetzten Werkzeuge endgültig festgedreht, so daß eine unverrückbare Lage der Wirbel 90, 90' gewährleistet ist. So kann, wie in Fig. 9 dargestellt, in der jeweiligen Stange 102, 104 ein entsprechendes Werkzeug vorhanden sein, es sind am oberen Ende die entsprechenden Sechskante ersichtlich, an die ein Drehwerkzeug angesetzt werden kann.

Nachdem das Repositionsgerät 100 abgenommen wurde, wird mit einem Imbusschlüssel in den ersten zylindrischen Abschnitt 18 des stabförmigen Körpers 22 eingefahren, und die Schraube 14 wird gelöst, also diese wird aus dem Innengewinde 80 der Fixierschraube 76 ausgedreht. Dabei bleibt der Sitz der Fixierschraube 76 unverändert.

Anschließend werden die Manipulatoren 10 axial abgezogen, wobei das einfach dadurch möglich ist, daß das entsprechende Werkzeug über die entsprechenden Rastmittel auf den jeweiligen Manipulator 10 aufgesetzt werden kann und dieser nunmehr vom jeweiligen Kopf der Pedikelschraube abgezogen wird.

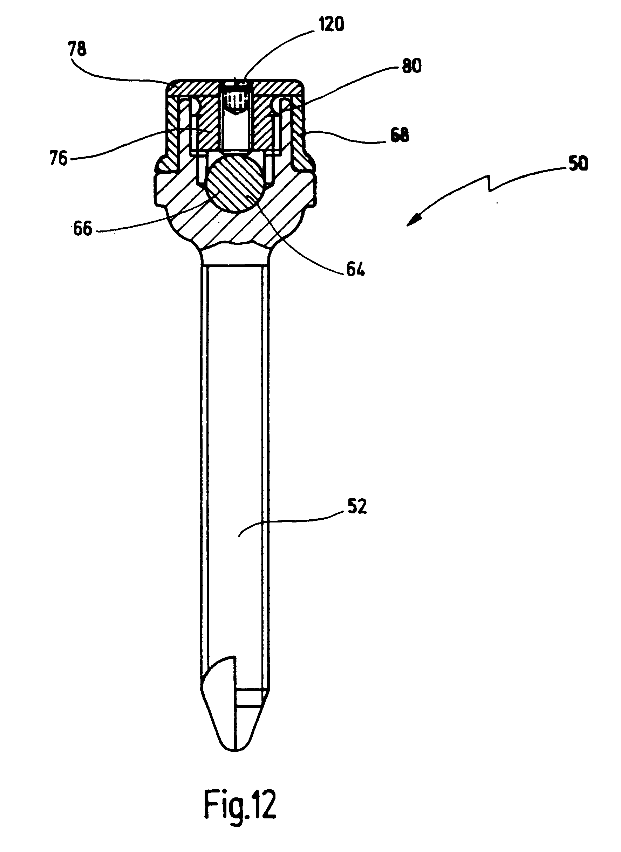

In das Innengewinde 80 der Fixierschraube 76, in der zuvor die Schraube 14 des Manipulators 10 eingedreht wird, wird nun abschließend eine Madenschraube 120 eingedreht, wie das aus Fig. 12 ersichtlich ist. Diese Madenschraube 180 wird soweit eingedreht, bis diese auf dem Stab 66 anliegt, somit für einen weiteren Fixierpunkt sorgt, so daß eine Drei-Punkt-Fixierung des Stabes 66 im Kopf der Pedikelschraube 50 sichergestellt ist. Die Länge der Madenschraube 120 ist dann so gewählt, daß ein ebener fluchtender Abschluß an der Oberseite der fertig implantierten Pedikelschraube 50 entsteht.

Der Preßring 68 und die Fixierschraube 76 können als relativ kleine und niedrig bauende Bauteile ausgebildet sein, die lediglich gewährleisten müssen, daß über die Drei-Punkt-Fixierung ein fester unverrückbarer Sitz des Stabes 66 am Kopf 56 der Pedikelschraube 50 sichergestellt ist. Die wesentlich höheren Kräfte beim Korrigieren und Setzen wurden durch den Manipulator 10 übertragen, der aber nun zwischenzeitlich abgenommen ist.

Dadurch ist dann ein Low-Profile-Design des Schraubkopfes möglich.

In Fig. 9 ist dargestellt, daß zwei jeweils auf derselben Seite liegende Pedikelschrauben 50', 50" durch einen Stab 66 verbunden sind.

Manchmal ist es wünschenswert, auch gegenüberliegende Pedikelschrauben miteinander zu verbinden, beispielsweise die Pedikelschraube 50' mit der Pedikelschraube 50.

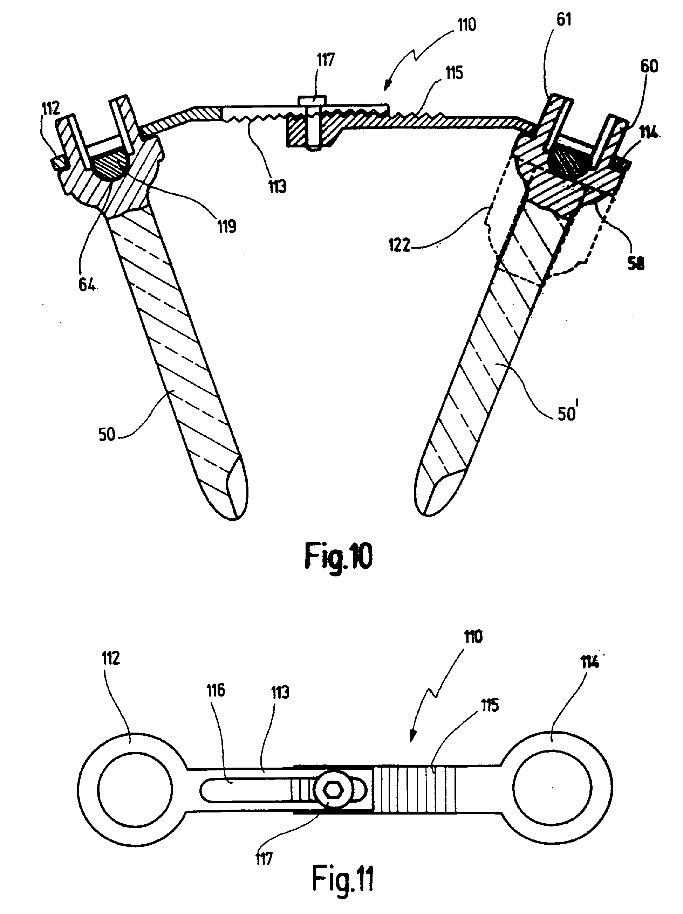

Aus den Fig. 10 und 11 ist nun ersichtlich, daß dazu ein Querverbinder 110 vorgesehen sein kann.

Der Querverbinder 110 besteht aus einem Ring 112 und einer ersten Zahnleiste 113, die mit einer entsprechenden Zahnleiste 115 zusammenwirkt, an deren äußerem Ende ein Ring 114 vorgesehen ist.

In der Zahnleiste 113 ist ein Langloch 116 vorgesehen, in dem verschiebbar eine Feststellschraube 117 sitzt, die über ein Gewinde 118 an der anderen Leiste 115 zum Feststellen einer Relativposition zwischen den kämmenden Zahnleisten 115 und 117 dient.

Bei der Handhabung wird nach Setzen der entsprechenden Pedikelschrauben 50' und 50 nach Einlegen eines Stabes 119 der Querverbinder 110 so aufgeschoben, daß dessen Ring 112 bzw. 114. über die hochstehenden Zylindermantelabschnitte 60 bzw. 61 geschoben werden. Die Querschnittskontur des Stabes 119 ist so, daß er einerseits in der entsprechenden halbrunden Mulde anliegt, dieser aber oben so abgeflacht ist, daß der jeweilige Ring 112 bzw. 114 flächig auf diesem aufliegt und dadurch die Zwei-Punkt-Fixierung bewerkstelligt. Anschließend wird ein entsprechender Preßring aufgeschoben und anschließend die ganze Sache mit der Fixierschraube 76 unter Zuhilfenahme eines Manipulators 10 wie zuvor beschrieben fixiert. Sollten aufgrund von Anomalien oder entsprechenden Positionen der Pedikelschrauben 50' bzw. 50 unterschiedliche Sitzhöhen der Köpfe resultieren, kann noch eine Distanzhülse 122 zwischengeschoben werden, wie das in Fig. 10 auf der rechten Seite durch gestrichelte Linien dargestellt wurde, um diese Höhenunterschiede auszugleichen.

## Patentansprüche

1. Manipulator mit einem stabförmigem Körper (12), wobei der Manipulator zur Handhabung einer eine Fixierschraube (76) aufweisenden Pedikelschraube (50, 50', 50'', 50''') in einem Wirbelkörper (90, 91, 92) einer Wirbelsäule dient, wobei ein Kopf (78) der Fixierschraube (76) konturiert ist,
der stabförmige Körper (12) eine Schraube (14) aufweist,
der stabförmige Körper (12) ein distales Ende (16) aufweist, das konturiert ist, so daß das distale Ende (16) an den konturierten Kopf (78) der Fixierschraube (76) ansetzbar und drehschlüssig und lösbar mit dieser verbindbar ist,
der stabförmige Körper (12) mittels dessen Schraube (14) zeitweilig mit der Fixierschraube (78) verschraubbar ist, und
wobei der Manipulator Kupplungsmittel zum Kuppeln des stabförmigen Körpers (12) mit einem stabförmigen Werkzeug (84, 100) aufweist,
**dadurch gekennzeichnet, daß** die Schraube (14) im Innern des stabförmigen Körpers (12) aufgenommen ist, und
daß im stabförmigen Körper (12) ein Gewinde (36) eingeschnitten ist, in das ein Gewinde (38) der Schraube (14) eindrehbar und durch dieses hindurch drehbar ist.

2. Manipulator nach Anspruch 1, **dadurch gekennzeichnet, daß** in der Fixierschraube (76) ein Gewinde (88) vorhanden ist, in das das Gewinde (38) der Schraube (14) eindrehbar ist.

3. Manipulator nach Anspruch 1 oder 2, **dadurch gekennzeichnet, daß** die Kupplungsmittel einen Mehrkant aufweisen, der mit einem entsprechenden Mehrkant am stabförmigen Werkzeug (84, 100) in kuppelndem Eingriff steht, wenn das Werkzeug (34, 100) auf dem stabförmigen Körper (12) aufgeschoben ist.

4. Manipulator nach Anspruch 3, **dadurch gekennzeichnet, daß** der Mehrkant als Außensechskant (22) am stabförmigen Körper (12) ausgebildet ist.

5. Manipulator nach einem der Ansprüche 1 bis 4, **dadurch gekennzeichnet, daß** die Kupplungsmittel Rastmittel aufweisen, um den Manipulator (10) mit dem aufgesetzten stabförmigen Werkzeug (84, 100) zu verrasten.

6. Manipulator nach Anspruch 5, **dadurch gekennzeichnet, daß** die Rastmittel als Kugelraste (87) ausgebildet sind.

7. Manipulator nach Anspruch 6, **dadurch gekennzeichnet, daß** die Rastmittel eine Aussparung (48) im stabförmigen Körper (12) aufweisen, in die eine federbelastete Kugel (87) des Werkzeuges (84, 100) einrastbar ist.

8. Manipulator nach einem der Ansprüche 1 bis 7, **dadurch gekennzeichnet, daß** der stabförmige Körper (12) im distalen Endbereich einen ersten zylindrischen Abschnitt (18) aufweist, an den sich nach proximal ein Außensechskant (22) anschließt, dessen Kreisumfangslinie einen geringeren Durchmesser (24) als der erste zylindrische Abschnitt (18) aufweist, und daß sich daran ein zweiter zylindrischer Abschnitt (26) mit noch geringerem Durchmesser (28) anschließt.

## Claims

1. Manipulator with a rod-shaped body (12), the manipulator serves for manoeuvering a pedicle screw (50, 50', 50 " , 50"') having a fixing screw (76) within a vertebral body (90, 91, 92) of a spinal column, a head (78) of the fixing screw (76) being contoured,
the rod-shaped body (12) has a screw (14),
the rod-shaped body (12) has a distal end (16), which is contoured in that the distal end (16) can be placed on the contoured head (78) of the fixing screw and can be connected in a rotationally fixed manner and releasably thereon,
the rod-shaped body (12) can be screwed via its screw (14) with the fixing screw (78) temporary, and
the manipulator has coupling means for coupling the rod-shaped body (12) to a rod-shaped tool (84, 100),
**characterized in that** the screw (14) is accommodated in the inside of the rod-shaped body (12), and
**in that** a thread (36) is cut in the rod-shaped body (12) into which thread (36) a thread (38) of the screw (14) can be turned in and screwed right through it.

2. Manipulator of claim 1, **characterized in that** a thread (88) is present in the fixing screw (76), and the thread (38) of the screw (14) can be turned into this thread (88).

3. Manipulator of claim 1 or 2, **characterized in that** the coupling means have a polygon which is in coupling engagement with a corresponding polygon on the rod-shaped tool (84, 100), when the tool (84, 100) is pushed onto the rod-shaped body (12).

4. Manipulator of claim 3, **characterized in that** the polygon is designed as an external hexagon (22) on the rod-shaped body (12).

5. Manipulator of anyone of claims 1 through 4, **characterized in that** the coupling means have locking means in order to lock the manipulator (10) to the applied rod-shaped tool (84, 100).

6. Manipulator of claim 5, **characterized in that** the locking means are designed as a ball catch (87).

7. Manipulator of claim 6, **characterized in that** the locking means have a recess (48) in the rod-shaped body (12), into which recess (48) a spring-loaded ball (87) of the tool (84, 100) can be locked.

8. Manipulator of anyone of claims 1 through 7, **characterized in that** the rod-shaped body (12) has, in the distal end area, a first cylindrical portion (18) which is adjoined proximally by an external hexagon (26) whose circle circumference line has a smaller diameter (24) than the first cylindrical portion (18), and **in that** the external hexagon (22) is adjoined by a second cylindrical portion (26) with a still smaller diameter (28).

## Revendications

1. Manipulateur avec un corps (12) en forme de tige, le manipulateur servant à manipuler une vis pédiculaire (50, 50', 50", 50"') présentant une vis de fixation (76) dans un corps de vertèbre (90, 91, 92) d'une colonne vertébrale, une tête (78) de la vis de fixation (76) étant profilée,
le corps (12) en forme de tige présentant une vis (14)
le corps (12) en forme de tige présentant une extrémité distale (16) qui est profilée, de sorte que l'extrémité distale (16) peut être rapportée sur la tête profilée (78) de la vis de fixation (76) et pouvant être reliée à celle-ci de manière solidaire en rotation et amovible,
le corps (12) en forme de tige pouvant se visser temporairement à la vis de fixation (78) au moyen de sa vis (14), et
le manipulateur présentant des moyens de couplage pour coupler le corps (12) en forme de tige à un outil (84, 100) en forme de tige,
**caractérisé en ce que** la vis (14) est logée à l'intérieur du corps (12) en forme de tige, et
**en ce que** dans le corps (12) en forme de tige est taillé un filet (36) dans lequel un filet (38) de la vis (14) peut se visser et traverser par rotation.

2. Manipulateur selon la revendication 1, **caractérisé en ce que** dans la vis de fixation (76) se trouve un filet (88) dans lequel peut se visser le filet (38) de la vis (14).

3. Manipulateur selon la revendication 1 ou 2, **caractérisé en ce que** les moyens de couplage présentent un polygone qui est en prise de couplage avec un polygone correspondant de l'outil (84, 100) en forme de tige quand l'outil (84, 100) est enfoncé sur le corps (12) en forme de tige.

4. Manipulateur selon la revendication 3, **caractérisé en ce que** le polygone est conformé en hexagone extérieur (22) sur le corps (12) en forme de tige.

5. Manipulateur selon l'une des revendications 1 à 4, **caractérisé en ce que** les moyens de couplage présentent des moyens d'encliquetage afin d'encliqueter le manipulateur (10) avec l'outil (84, 100) en forme de tige mis en place.

6. Manipulateur selon la revendication 5, **caractérisé en ce que** les moyens d'encliquetage sont conformés en cliquet à bille (87).

7. Manipulateur selon la revendication 6, **caractérisé en ce que** les moyens d'encliquetage présentent un évidement (48) dans le corps en forme de tige dans lequel peut s'encliqueter une bille (87) montée sur ressort de l'outil (84, 100).

8. Manipulateur selon l'une des revendications 1 à 7, **caractérisé en ce que** le corps (12) en forme de tige présente dans la zone d'extrémité distale une première section cylindrique (18) qui est suivi, du côté proximal, par un hexagone extérieur (12) dont la ligne de pourtour circulaire présente un diamètre inférieur à celui de la première section cylindrique (18), et **en ce qu'**à la suite se trouve une deuxième section cylindrique (26) de diamètre (28) encore plus petit.
